# EUROPÄISCHE PATENTSCHRIFT

(11) **EP 3 720 678 B1**
(45) Veröffentlichungstag und Bekanntmachung des Hinweises auf die Patenterteilung: **08.06.2022**
(21) Anmeldenummer: 19715413.1
(22) Anmeldetag: 22.03.2019
(51) Int. Cl.: B29C 45/36, A61B 6/00, G21K 1/02

(54) **VERFAHREN ZUM HERSTELLEN EINES STRAHLLEITRASTERS SOWIE NACH DEM VERFAHREN HERGESTELLTES STRAHLLEITRASTER**
METHOD FOR PRODUCING A BEAM GUIDE GRID AND A BEAM GUIDE GRID PRODUCED IN ACCORDANCE WITH THE METHOD
PROCÉDÉ DE FABRICATION D'UNE GRILLE FORMANT GUIDE DE RAYONNEMENTS AINSI QUE GRILLE FORMANT GUIDE DE RAYONNEMENTS RÉALISÉE SELON CE PROCÉDÉ

(30) Priorität: 04.04.2018 DE 102018107969
(43) Veröffentlichungstag der Anmeldung: 14.10.2020
(73) Patentinhaber: Leonhardt E.K., 73269 Hochdorf (DE)
(72) Erfinder: LEONHARDT, Wolfgang, 73269 Hochdorf (DE); SCHMORL, Gunnar, 73033 Göppingen (DE)
(74) Vertreter: Fleck, Hermann-Josef
(86) Internationale Anmeldenummer: PCT/EP2019/057253
(87) Internationale Veröffentlichungsnummer: WO 2019/192859

(56) Entgegenhaltungen:
- EP-A2- 1 182 671
- DE-A1-102004 027 158
- JP-A- 2003 251 658

## Beschreibung

Die Erfindung bezieht sich auf ein Verfahren zum Herstellen eines Strahlleitrasters, das aus einem mit einem Raster aus Durchgangskanälen und diese umgebenden Wandbereichen versehenen Formkörper aus einer Mischung von strahlungsabsorbierendem Metallpulver und Bindemittel, insbesondere Wolframpulver und Bindemittel, hergestellt wird, sowie auf ein nach dem Verfahren hergestelltes Strahlleitraster.

Derartige Strahlleitraster, auch als Streustrahlenraster oder Kollimator bezeichnet, werden insbesondere in der Röntgen- oder Gammastrahlendiagnostik, beispielsweise in Computertomographie-Anlagen, verwendet, um störende Streustrahlung, die sich bei der Bildung eines zu untersuchenden Objekts den primären Abbildungsstrahlen überlagern, möglichst weitgehend zu absorbieren und eine möglichst störungsfreie Abbildung auf einer nachfolgenden Empfängeranordnung zu erzielen und ein möglichst gut auswertbares Bild erzeugen zu können. Derartige Lichtleitraster besitzen von dünnen Wandbereichen z. B. im Bereich von 100 µm Dicke umgebene Durchgangskanäle mit z. B. einer Öffnungsweite von näherungsweise 1 mm oder kleiner, die mit ihrer zentralen Durchgangsachse auf einen gemeinsamen Fokuspunkt gerichtet sind, in dem die Strahlungsquelle angeordnet ist. Dadurch ergibt sich eine Schrägausrichtung der individuellen Durchgangskanäle relativ zueinander, woraus Schwierigkeiten bei der Fertigung derartiger Strahlleitraster resultieren. Die Wandbereiche sind aus einem die Streustrahlung möglichst weitgehend absorbierenden Material hergestellt, das z. B. Wolfram oder ein Metall mit vergleichbarer Absorptionsfähigkeit für die Röntgen- beziehungsweise Gammastrahlung enthält.

Ein Verfahren zur Herstellung eines Strahlleitrasters und ein nach diesem Verfahren hergestelltes Strahlleitrasters der eingangs genannten Art zeigt die DE 10 2004 027 158 A1. Das Strahlleitraster oder der Kollimator ist aus absorbierendem Material zum Unterdrücken von Streustrahlen beispielsweise in Spritzgusstechnik hergestellt. Bei einem Ausführungsbeispiel werden zwei Grundkörper übereinander gestapelt und mit Schnappverschlüssen aneinander befestigt. Es wird z. B. ein zellenartiges Streustrahlenraster gebildet, mit dem eine Kollimierung z. B. in zwei verschiedene Richtungen erreicht wird.

Ein weiteres Verfahren zum Herstellen eines Strahlleitrasters ist in der EP 1 298 678 A2 angegeben. Bei diesem bekannten Verfahren wird ein Grundkörper entsprechend der Durchgangskanäle beziehungsweise durchlässigen Bereiche oder der undurchlässigen Wandbereiche mittels einer Rapid-Prototyping-Technik durch schichtweise Verfestigung eines Aufbaumaterials unter Einwirkung von Strahlung aufgebaut und auf Basis dieses Grundkörpers wird das Strahlleitraster beziehungsweise Streustrahlenraster oder der Kollimator fertiggestellt. Dazu wird beispielsweise in Zwischenräume des Grundkörpers das die Streustrahlung absorbierende Material in flüssigem Zustand eingefüllt und durch Abkühlen verfestigt. Das Material des Grundkörpers kann anschließend entfernt werden, so dass lediglich das Gerüst aus dem absorbierenden Material als Strahlleitraster verbleibt. Bei einer anderen Vorgehensweise wird der Grundkörper in eine Negativform, beispielsweise durch Einfüllen oder Eingießen von Nickel, abgeformt, und anschließend kann ein Strahlleitraster aus dieser Negativform in vorstehend beschriebener Weise erstellt werden. Diese Beispiele zeigen, dass für die Herstellung eines Strahlleitrasters hoher Aufwand erforderlich ist.

In der US 6 470 072 B1 und der mit ihr inhaltlich zusammenhängenden EP 1 182 671 A2 ist vorgeschlagen, auf verschiedene Brennweiten einstellbare Strahlleitraster bereitzustellen, indem diese flexibel ausgebildet werden. Für die Herstellung ist ein Spritzgießen vorgeschlagen, wobei eine Mischung aus Wolframpulver und thermoplastischem Material genannt ist. Nähere Angaben, wie die Herstellung im Einzelnen erfolgen soll, insbesondere wie die vorstehend genannten Schwierigkeiten zu überwinden sind, sind nicht gemacht.

Die JP 2003-251 658 A zeigt die Herstellung eines Lichtleitkörpers mit feinen Poren mittels Spritzgießens, wobei eine Form mit beweglicher Teilform verwendet wird, die von einer Gruppe paralleler Stifte verschieblich durchdrungen wird. Mit einer solchen Vorrichtung wird jedoch kein fokussierendes Raster erhalten.

In der US 2012/0085942 A1 ist ein Verfahren zum Herstellen von Strahlleitrastern bzw. Kollimatoren offenbart, bei dem aus Wolframpulver Teilkörper durch Sintern gefertigt werden, die zu den Kollimatoren zusammengesetzt werden.

In der US 2010/0276829 A1 ist die Herstellung unter anderem von Streustrahlenrastern mit hohem Aspekt-Verhältnis durch Formen einer Formmasse mit z. B. 80 Gew.-% bis 98 Gew. -% Pulvermaterial, wie Wolframpulver, und Binder in einer Form dargestellt.

Die DE 10 2011 050 963 A1 zeigt ein Verfahren zur Herstellung eines Anti-Streu-Röntgengitters, bei dem ein Substrat mit Kanälen bereitgestellt und anschließend auf den Seitenwänden der Kanäle mit einem Material beschichtet wird, das Röntgenstrahlen nicht absorbiert, und darauf mit einem Material, das Röntgenstrahlen absorbiert. Als Herstellungsverfahren für das Substrat sind ohne nähere Ausführungen Spritzguss, Lasern, mechanische Bearbeitung, Plasmaätzen und dergleichen genannt, wobei als Substratmaterial, das ebenfalls für Röntgenstrahlen nicht absorbierend ist, Thermoplaste, PEEK, Grafit, Aluminium und Kombinationen davon genannt sind. Die axiale Ausrichtung der Kanäle kann dem aus der Quelle austretenden Röntgenstrahlkonus entsprechen.

Die US 2013/0193329 A1 zeigt eine Neutronen-Szintillator-Zusammensetzung aus einem Neutronen-Szintilator und einem Binder. In der US 5 034 157 ist eine für Spritzgießen geeignete Mixtur offenbart, die ein Polyetherketon-Matrixmaterial, wie PEEK-Harz, enthält.

In der US 7 839 981 B2 ist ein Strahlleitraster aus verschiedenen Materialien und eine entsprechende Herstellungsweise gezeigt.

Der vorliegenden Erfindung liegt die Aufgabe zugrunde, ein Verfahren zum Herstellen eines Strahlleitrasters der vorstehend beschriebenen Art anzugeben, mit welchem insbesondere große Stückzahlen möglichst ökonomisch herstellbar sind. Auch sollen entsprechend ökonomisch hergestellte Strahlleitraster sowie deren Verwendung bereitgestellt werden.

Diese Aufgabe wird bezüglich des Verfahrens durch die im Anspruch 1 genannte Vorgehensweise gelöst. Hinsichtlich des Strahlleitrasters wird die Aufgabe mit den im Anspruch 7 angegebenen Merkmalen gelöst. Hinsichtlich der Anordnung und Anwendung wird die Aufgabe mit den Merkmalen der Ansprüche 8 beziehungsweise 9 gelöst.

Bei dem Verfahren ist also in Verbindung mit den Merkmalen des Oberbegriffs vorgesehen, dass der Formkörper mittels Spritzgießens hergestellt wird, wobei die homogenisierte Mischung als fließfähig aufbereitete Spritzmasse mittels einer Spritzmaschine in ein den Formkörper formendes Formwerkzeug gefüllt wird, in das vor dem Einfüllen der Spritzmasse bewegbare Formkerne eingeführt wurden.

Mit diesen Maßnahmen, die sich im Rahmen des Spritzgießens ebenso auf ein Spritzprägeverfahren beziehen können, wird mit einer entsprechend aufgebauten Form eine ökonomische Herstellung insbesondere hoher Stückzahlen erreicht. Dabei lassen sich mit gleichbleibender Qualität hochwertige Strahlleitraster fertigen, wobei insbesondere auch weitgehend störungsfreie Innenwandflächen an den Wandbereichen erhalten werden.

Entsprechende Vorteile ergeben sich auch für die so hergestellten Strahlleitraster und die daraus gebildete Anordnung beziehungsweise die darauf basierende Anwendung.

Verschiedene Ausgestaltungen des Verfahrens bestehen darin, dass als Bindemittel ein für den Spritzvorgang vorgewärmter und plastifizierter thermoplastischer oder ein duroplastischer Kunststoff verwendet wird.

Eine für die Durchführung und die Qualität der erhaltenen Strahlleitraster vorteilhafte weitere Maßnahme besteht darin, dass als thermoplastischer Kunststoff ein Polyetherketon, insbesondere Polyetheretherketon (PEEK), verwendet wird.

Für die Durchführung des Verfahrens und die Funktion der Strahlleitraster sind des Weiteren die Maßnahmen von Vorteil, dass das Verhältnis von Metallpulver, insbesondere Wolframpulver, zu Bindemittel in Volumenprozent im Bereich von 30/70 bis 98/2 liegt. Beispielsweise haben sich dabei Bereiche von 40/60 bis 95/5, von 50/50 bis 90/10, insbesondere 60/40 bis 85/15 oder auch verschiedene Bereiche innerhalb dieser Bereichsgrenzen in ersten Untersuchungen der Erfinder als vorteilhaft gezeigt.

Die Durchführung des Verfahrens kann vorteilhaft in der Weise erfolgen, dass beim Formungsvorgang - um zu erreichen, dass die Achsen der Durchgangskanäle unter spitzem Winkel zueinander auf einen gemeinsamen Fokuspunkt gerichtet sind - die Formkerne in dem Formwerkzeug entsprechend der Orientierung, dem Raster und der Form der Durchgangskanäle angeordnet und gestaltet sind, welche außerhalb eines Wandabschnitts des Formwerkzeugs an einer Lagereinheit gehalten werden und nach dem Einfüllen und einem Vorverfestigen der Spritzmasse und vor einem Auswerfen des Formkörpers aus den Durchgangskanälen herausgezogen werden.

Ferner sind für die Durchführung des Verfahrens die Maßnahmen von Vorteil, dass die Formkerne zur Vorbereitung des Formungsvorgangs mittels einer die Lagereinheit mit den Formkernen bewegenden Verstelleinheit in Formungsposition gebracht werden und nach dem Vorverfestigen mittels der die Lagereinheit bewegenden Verstelleinheit aus den Durchgangskanälen herausgezogen werden.

Weitere Vorteile für die Durchführung des Verfahrens ergeben sich dadurch, dass die Formkerne durch individuell zugeordnete Durchführungen in dem Wandabschnitt des Formwerkzeugs eingeführt sind und durch diese herausgezogen werden, wobei sie an der Lagereinheit schwimmend gehalten werden, um eine sich durch einen Schrägverlauf der Durchgangskanäle und Durchführungen beim Einführen und Herausziehen bewirkte seitliche Bewegungskomponente zumindest in x- und/oder y-Richtung, gegebenenfalls aber auch in z-Richtung auszugleichen.

Das Strahlleitraster ist erfindungsgemäß so aufgebaut, dass die Durchgangskanäle mit ihren zentralen Achsen bezogen auf einen gemeinsamen Fokuspunkt in spitzem Winkel schräg zueinander ausgerichtet sind.

Eine präzise Ausbildung der Strahlleitraster wird erfindungsgemäß ferner dadurch erreicht, dass die Durchgangskanäle sich von einer Strahleinfallsseite zu einer Strahlausfallseite hin konisch erweitern, wobei der Konizitätswinkel gegenüber der zentralen Achse höchstens 1 ° beträgt.

Weitere erfindungsgemäße Merkmale der Strahlleitraster bestehen darin, dass der Rasterabstand von Mitte zu Mitte der Durchgangskanäle höchstens 2 mm und die Dicke der Wandbereiche an ihrer dünnsten Stelle höchstens 200 µm beträgt.

Die Erfindung wird nachfolgend anhand von Ausführungsbeispielen unter Bezugnahme auf die Zeichnungen näher erläutert. Es zeigen:
- Fig. 1: eine schematische Darstellung zum Einsatz eines Strahlleitrasters in einem Röntgenbildaufnahmegerät, wie Computertomograph,
- Fig. 2: eine vergrößerte ausschnittsweise Darstellung eines Strahlleitrasters im Längsschnitt,
- Fig. 3: eine ausschnittsweise Draufsicht eines Strahlleitrasters in schematischer Darstellung und
- Fig. 4: ein Spritzwerkzeug zur Herstellung eines Strahlleitrasters in einer perspektivischen Schnittansicht in schematischer Darstellung.

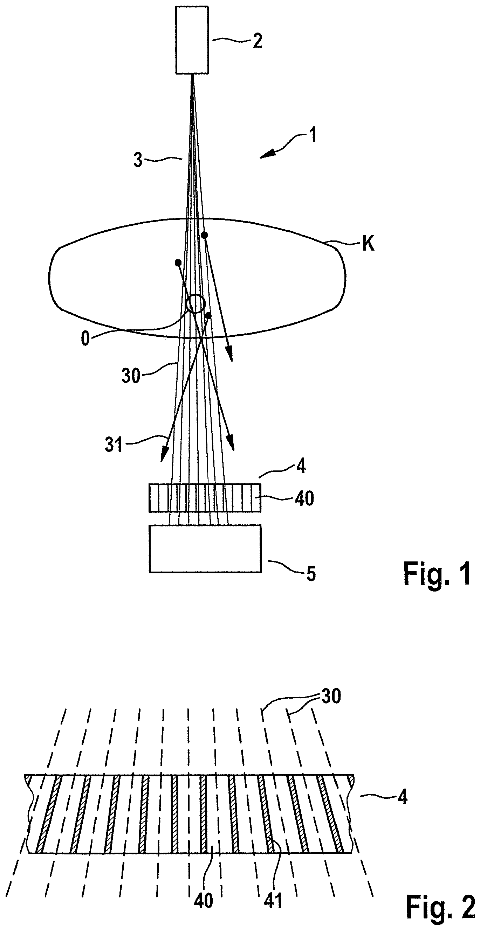

In Fig. 1 ist der Einsatz eines Strahlleitrasters, auch als Streustrahlenraster oder Kollimator bezeichnet, in einem Aufnahmegerät 1 der Röntgendiagnostik gezeigt. In einem Fokuspunkt ist z. B. in einem Abstand von einem Meter eine Strahlungsquelle 2 zur Abgabe von Röntgenstrahlen angeordnet, die einen Körper K mit einem zu untersuchenden Objekt O durchdringen. Anschließend durchlaufen die Röntgenstrahlen, die die Bildinformation des Objekts O enthalten, als Primärstrahlen 30 Durchgangskanäle 40 des Strahlleitrasters 4 und werden von einer Empfängereinheit 5 aufgenommen, die in der Regel matrixartig angeordnete Empfängerelemente aufweist. Anschließend wird aus den Einzelaufnahmen in an sich bekannter Weise ein auswertbares Bild des Objekts O rekonstruiert. In ähnlicher Weise kann auch eine Untersuchung mit Gammastrahlen, das heißt mit Strahlen eines sich an den kürzeren Wellenlängenbereich der Röntgenstrahlen anschließenden Bereichs elektromagnetischer Strahlung, erfolgen. Dann ist die Strahlungsquelle 2 als Gammastrahlungsquelle ausgebildet und die Empfängereinheit 5 entsprechend ausgelegt.

Wie in Fig. 1 weiter schematisch dargestellt, können sich z. B. an in dem Körper K enthaltenen Streuzentren beim Durchtritt des Strahlenbündels 3 Streustrahlen 31 ergeben, die das durch die Primärstrahlen 30 erhaltene Bild stören beziehungsweise verrauschen, so dass die Auswertung der Bildinformation erschwert ist oder nicht mehr detailgenau erfolgen kann. Mittels des Strahlleitrasters werden Streustrahlen weitgehend absorbiert, wozu die Durchgangskanäle 40 des Strahlleitrasters 4 mit absorbierenden Wandbereichen 41 umgeben sind. Geeignete absorbierende Materialien, die betreffende Metalle, wie z. B. Wolfram oder Metalle mit ähnlichen absorbierenden Eigenschaften für die Röntgenstrahlung beziehungsweise Gammastrahlung enthalten, sind an sich bekannt.

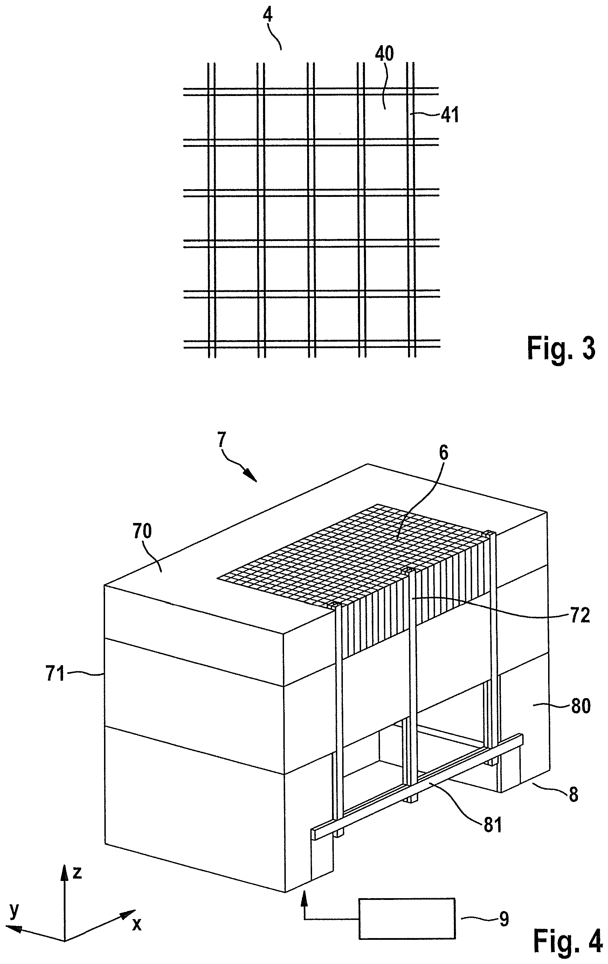

Damit die Primärstrahlen 30 ungehindert durch das Strahlleitraster 4 treten können, sind die Durchgangskanäle 40 mit ihren absorbierenden Wandbereichen auf den in der Strahlungsquelle 2 angeordneten Fokuspunkt hin ausgerichtet, wie in der Ausschnittsdarstellung nach Fig. 2 angedeutet ist. Wie aus Fig. 3 ersichtlich, ist das Strahlleitraster 4 zweidimensional ausgedehnt und beispielsweise mit im Querschnitt rechteckförmigen, quadratischen, anderer Mehreckform, runden, ovalen oder in geeigneter Weise anders frei geformten Durchgangskanälen 40 versehen.

Das Aufnahmegerät 1 besitzt eine Anordnung aus einer Vielzahl nebeneinander angeordneter Strahlleitraster 4, um ein möglichst vollständiges Bild des Objekts O zu erhalten. Beispielsweise haben die einzelnen Strahlleitraster 4 eine Höhe (bezüglich des Strahldurchtritts) zwischen 4 und 20 mm und eine Länge und Breite von einigen Zentimetern und die Querschnittsausdehnung der Durchgangskanäle liegt z. B. im Bereich um einen Millimeter, z. B. zwischen 0,5 und 1,5 mm. Die Stärke der Wandbereiche beträgt dabei z. B. zwischen 40 µm und 200 µm, beispielsweise zwischen 60 µm und 150 µm, wobei für die Herstellung ein Bereich zwischen z. B. 80 µm und 120 µm geeignet sein kann. Die Schrägstellung der Durchgangskanäle 40 bezieht sich z. B. auf einen in einer Entfernung von einem Meter entfernten gemeinsamem Fokuspunkt, so dass die einzelnen Durchgangskanäle 40 bezüglich des Fokuspunkts in einem spitzen Winkel zueinander verlaufen.

Wie eingangs beschrieben, führen die Anforderungen an die genannte Ausbildung des Strahlleitrasters 4 zu einem relativ hohen Aufwand. Um den Aufwand insbesondere hinsichtlich großer Stückzahlen und gleichmäßig guter Bearbeitung ökonomisch zu gestalten, werden die Strahlleitraster 4 erfindungsgemäß spritzgießtechnisch hergestellt, wobei der Aufbau und die Vorgehensweise durch besondere Maßnahmen gekennzeichnet sind, wie anhand von Fig. 4 schematisch dargestellt ist.

Fig. 4 zeigt ein Spritzwerkzeug, welches in einer Spritzgießmaschine am Ausgang vor einer eine Spritzmasse einspritzenden Spritzdüse angeordnet ist. Das Spritzwerkzeug weist ein Formwerkzeug 7 mit einer Kavität für einen das spätere Strahlleitraster 4 bildenden Spritzkörper beziehungsweise Formkörper 6 auf. Ein plattenförmiges Formteil 70 ist düsenseitig angeordnet und bildet die äußere Umfangskontur des Formkörpers 6. Unter dem Formteil 70 befindet sich auswerferseitig ein weiteres plattenförmiges Formteil 71. In dem auswerferseitigen weiteren Formteil 71 sind Durchführöffnungen angeordnet, durch die verfahrbare Formkerne 72 in Richtung der auch in dem Formkörper 6 vorhandenen Durchgangskanäle 40 geführt werden. In Fig. 4 sind nur ein paar wenige dieser stiftartigen Formkerne 72 dargestellt. Tatsächlich sind derartige Formkerne 72 für alle Durchgangskanäle 40 vorhanden.

Jeder der Formkerne 72 ist individuell in dem bezüglich des Fokuspunkts erforderlichen spitzen Winkel zu einer senkrechten Achse (z-Achse) des Strahlleitrasters 4 beziehungsweise des Formkörpers 6 ausgerichtet, wobei das Strahlleitraster 4 beziehungsweise der Formkörper 6 sich in Länge und Breite in einer zur z-Richtung rechtwinklig liegenden Ebene in x-/y-Richtung erstreckt. Bei in die Kavität eingeführten Formkernen 72 wird eine Spritzmasse zum Herstellen des Formkörpers 6 über die betreffende Düse der Spritzgussmaschine eingeführt, um die absorbierenden Wandbereiche 41 herzustellen, wobei die Spritzmasse durch eine Mischung von strahlungsabsorbierendem Metallpulver und Bindemittel, insbesondere also Wolframpulver und Bindemittel, gebildet ist.

Die erwärmte flüssige Spritzmasse wird nachfolgend auf eine Temperatur abgekühlt, die eine Vorverfestigung des Formkörpers 6 ergibt. Anschließend werden die Formkerne 72 aus den Durchgangskanälen des Formkörpers 6 mittels eines die Formkerne 72 tragenden Haltekörpers 80 unter Betätigung durch eine Verstelleinheit 9 herausgezogen. Hierzu weist der Haltekörper 80 Formkernhalter 81 auf, die während des Herausziehens der Formkerne 72 aus dem Formkörper 6 eine bezüglich der Z-Richtung seitliche Verstellung ermöglichen, da beim Herausziehen der Formkerne 72 in z-Richtung wegen des schrägen Verlaufs der Durchgangskanäle 40 sich den Formkernen 72 eine seitliche Bewegungskomponente in x- beziehungsweise y-Richtung überlagert. Die Lagerung der Formkerne 72 in den Formkernhaltern 81 ist daher schwimmend ausgebildet. Entsprechend erlaubt die schwimmende Ausbildung auch beim Einführen der Formkerne 72 durch die Durchführungen des auswerferseitigen weiteren Formteils 71 eine seitliche Verlagerung in x- und y-Richtung.

Wie Fig. 4 zeigt, sind für die schwimmende Lagerung z. B. einzelne horizontale, in x-Richtung verlaufende Führungen für eine jeweilige Zeile von Formkernen 72 vorhanden, während sich die Führungen mit den betreffenden Formkernen 72 in y-Richtung ihrerseits verstellen können, um auch in Spaltenrichtung eine entsprechende, auf den Fokuspunkt ausgerichtete Richtung der Durchgangskanäle 40 beim Einführen beziehungsweise Herausziehen der Formkerne 72 ausgleichen zu können.

Als vorteilhafte Maßnahme für das Entformen hat sich herausgestellt, dass die Formkerne 72 in Richtung zum Fokuspunkt hin leicht oder mehr oder weniger stark konisch verjüngt sind.

Die Öffnung des Spritzwerkzeugs beziehungsweise Formwerkzeugs 7 zum Auswerfen des Formkörpers 6 beziehungsweise Strahlleitrasters 4 erfolgt zwischen dem Formteil 70 und dem weiteren Formteil 71.

In Untersuchungen der Erfinder hat sich ergeben, dass als Bindemittel z. B. ein thermoplastischer Kunststoff aus Polyetherketon, insbesondere Polyetheretherketon (PEEK), vorteilhaft verwendet werden kann, welcher stabile Eigenschaften und eine Formbarkeit bei relativ hoher Temperatur (z. B. zwischen 330 °C und 450 °C) ermöglicht. Die Temperaturdifferenz zwischen der Temperatur beim Befüllen des Formwerkzeugs 7 und beim Herausziehen der Formkerne 72 beträgt dabei z. B. 70 °C, wozu die Temperiereinrichtung der Spritzgussmaschine entsprechend steuerbar ausgebildet ist. Denkbar sind auch andere thermoplastische Bindemittel oder auch duroplastische Bindemittel. Der Füllgrad der Spritzmasse, welche homogen mit Metallpulver aufbereitet ist, liegt in Volumenanteilen von Metallpulver beziehungsweise Wolframpulver zu Bindemittel im Bereich zwischen 30/70 bis 98/2 Teilen Metallpulver zu Bindemittel, wie z. B. zwischen 40/60 bis 95/5, 50/50 bis 90/10 oder 60/40 bis 85/15, wobei auch dazwischenliegende Verhältnisse in Frage kommen. Dabei sind auch die gewählten Materialien für das strahlungsabsorbierende Metallpulver und das Bindemittel sowie die geometrischen Verhältnisse des Strahlleitrasters, wie Dicke der Wandbereiche, zu berücksichtigen.

Das erfindungsgemäße Verfahren ergibt eine ökonomische Herstellungsweise von Strahlleitrastern 4 insbesondere bei hohen Stückzahlen und gleichbleibend hoher Qualität.

## Patentansprüche

1. Verfahren zum Herstellen eines Strahlleitrasters (4), das aus einem mit einem Raster aus Durchgangskanälen (40) und diese umgebenden Wandbereichen versehenen Formkörper aus einer Mischung von strahlungsabsorbierendem Metallpulver und Bindemittel, insbesondere Wolframpulver und Bindemittel, hergestellt wird, wobei der Formkörper mittels Spritzgießens hergestellt wird und dabei die homogenisierte Mischung als fließfähig aufbereitete Spritzmasse mittels einer Spritzmaschine in ein den Formkörper formendes Formwerkzeug (7) gefüllt wird,
**dadurch gekennzeichnet,**
**dass** in das Formwerkzeug (7) vor dem Einfüllen der Spritzmasse bewegbare Formkerne (72) eingeführt wurden und
**dass** beim Formungsvorgang - um zu erreichen, dass die Achsen der Durchgangskanäle (40) unter spitzem Winkel zueinander auf einen gemeinsamen Fokuspunkt gerichtet sind - die Formkerne (72) in dem Formwerkzeug entsprechend der Orientierung, dem Raster und der Form der Durchgangskanäle (40) angeordnet und gestaltet sind, welche außerhalb eines Wandabschnitts des Formwerkzeugs (7) an einer Lagereinheit (8) gehalten werden und nach dem Einfüllen und einem Vorverfestigen der Spritzmasse und vor einem Auswerfen des Formkörpers aus den Durchgangskanälen (40) herausgezogen werden.

2. Verfahren nach Anspruch 1,
**dadurch gekennzeichnet,**
**dass** als Bindemittel ein für den Spritzvorgang vorgewärmter und plastifizierter thermoplastischer oder ein duroplastischer Kunststoff verwendet wird.

3. Verfahren nach Anspruch 2,
**dadurch gekennzeichnet,**
**dass** als thermoplastischer Kunststoff ein Polyetherketon, insbesondere Polyetheretherketon (PEEK), verwendet wird.

4. Verfahren nach einem der vorhergehenden Ansprüche,
**dadurch gekennzeichnet,**
**dass** das Verhältnis von Metallpulver, insbesondere Wolframpulver, zu Bindemittel in Volumenprozent im Bereich von 30/70 bis 98/2 liegt.

5. Verfahren nach einem der vorhergehenden Ansprüche,
**dadurch gekennzeichnet,**
**dass** die Formkerne (72) zur Vorbereitung des Formungsvorgangs mittels einer die Lagereinheit (8) mit den Formkernen (72) bewegenden Verstelleinheit (9) in Formungsposition gebracht werden und nach dem Vorverfestigen mittels der die Lagereinheit (8) bewegenden Verstelleinheit (9) aus den Durchgangskanälen (40) herausgezogen werden.

6. Verfahren nach einem der vorhergehenden Ansprüche,
**dadurch gekennzeichnet,**
**dass** die Formkerne (72) durch individuell zugeordnete Durchführungen in dem Wandabschnitt des Formwerkzeugs (7) eingeführt sind und durch diese herausgezogen werden, wobei sie an der Lagereinheit (8) schwimmend gehalten werden, um eine sich durch einen Schrägverlauf der Durchgangskanäle (40) und Durchführungen beim Einführen und Herausziehen bewirkte seitliche Bewegungskomponente in x- und/oder y-Richtung auszugleichen.

7. Strahlleitraster (4), das aus einem Formkörper mit einem Raster aus Durchgangskanälen (40), die mit ihren zentralen Achsen bezogen auf einen Fokuspunkt in spitzem Winkel schräg zueinander ausgerichtet sind, und mit diese umgebenden, Röntgen- oder Gammastrahlen absorbierenden Wandbereichen gebildet ist, welche aus in einem Bindemittel gebundenem, homogen verteilten Metallpulver, insbesondere Wolframpulver, geformt sind, wobei das Bindemittel ein thermoplastischer oder duroplastischer Kunststoff ist oder überwiegend einen solchen Kunststoff enthält und der Formkörper mittels Spritzgießens hergestellt ist, indem die homogenisierte Mischung als fließfähig aufbereitete Spritzmasse mittels einer Spritzmaschine in ein den Formkörper formendes Formwerkzeug (7) gefüllt wird,
**dadurch gekennzeichnet,**
**dass** das Strahlleitraster (4) dadurch erhalten wird, dass in das Formwerkzeug (7) vor dem Einfüllen der Spritzmasse bewegbare Formkerne (72) eingeführt wurden und dass beim Formungsvorgang - um zu erreichen, dass die Achsen der Durchgangskanäle (40) unter spitzem Winkel zueinander auf einen gemeinsamen Fokuspunkt gerichtet sind - die Formkerne (72) in dem Formwerkzeug entsprechend der Orientierung, dem Raster und der Form der Durchgangskanäle (40) angeordnet und gestaltet sind, welche außerhalb eines Wandabschnitts des Formwerkzeugs (7) an einer Lagereinheit (8) gehalten werden und nach dem Einfüllen und einem Vorverfestigen der Spritzmasse und vor einem Auswerfen des Formkörpers aus den Durchgangskanälen (40) herausgezogen werden,
**dass** die Durchgangskanäle (40) sich von einer Strahleinfallsseite zu einer Strahlausfallseite hin konisch erweitern, wobei der Konizitätswinkel gegenüber der zentralen Achse höchstens 1° beträgt und
**dass** der Rasterabstand von Mitte zu Mitte der Durchgangskanäle (40) höchstens 2 mm und die Dicke der Wandbereiche an ihrer dünnsten Stelle höchstens 200 µm beträgt.

8. Anordnung aus einer Vielzahl nebeneinander angeordneter Strahlleitraster (4) des Aufbaus nach Anspruch 7, in einem mindestens eine Röntgenstrahlenquelle aufweisenden optischen System, bei dem die Durchgangskanäle (40) mit ihren zentralen Achsen auf die Röntgenstrahlenquelle ausgerichtet sind.

9. Anwendung von Strahlleitrastern des Aufbaus nach Anspruch 7 oder einer Anordnung nach Anspruch 8 in einem Computertomograph.

## Claims

1. Method for producing a beam guide grid (4) produced from a molded body which is provided with a grid of through-channels (40) and wall regions surrounding said channels and is made of a mixture of radiation-absorbing metal powder and binder, in particular tungsten powder and binder, the molded body being produced by means of injection molding, in which the homogenized mixture, as an injection molding compound which has been prepared so as to be flowable, is filled into a mold (7) which molds the molded body by means of an injection molding machine,
**characterized in that**
movable mold cores (72) are introduced into the mold (7) before the filling of the injection molding compound, and
**in that**, during the molding process, in order to ensure that the axes of the through-channels (40) are directed to a common focus point at an acute angle to one another, the mold cores (72) are arranged and formed in the mold in accordance with the orientation, the grid and the shape of the through-channels (40), which mold cores are held outside a wall portion of the mold (7) on a storage unit (8) and are removed from the through-channels (40) after filling and pre-solidification of the injection molding compound and before ejection of the molded body.

2. Method according to claim 1,
**characterized in that**
a thermoplastic plastics material which is preheated and plasticized for the injection molding process or a thermosetting plastics material is used as the binder.

3. Method according to claim 2,
**characterized in that**
a polyetherketone, in particular polyether ether ketone (PEEK), is used as the thermoplastic plastics material.

4. Method according to any of the preceding claims,
**characterized in that**
the ratio of metal powder, in particular tungsten powder, to binder in percent by volume is in the range from 30/70 to 98/2.

5. Method according to any of the preceding claims,
**characterized in that**
the mold cores (72) are brought into the molding position in preparation for the molding process by means of a movement unit (9) which moves the storage unit (8) with the mold cores (72), and, after pre-solidification, are removed from the through-channels (40) by means of the movement unit (9) which moves the storage unit (8).

6. Method according to any of the preceding claims,
**characterized in that**
the mold cores (72) are inserted through individually assigned feed-throughs in the wall portion of the mold (7) and are removed through same, said mold cores being held on the storage unit (8) in a floating manner in order to compensate for lateral movement components in the x and/or y direction caused by an oblique course of the through-channels (40) and feed-throughs during insertion and removal.

7. Beam guide grid (4) formed from a molded body having a grid of through-channels (40), the central axes of which are oriented obliquely to one another at an acute angle with respect to a focal point, and having wall regions surrounding said through-channels that absorb X-rays or gamma rays and are formed from homogeneously distributed metal powder, in particular tungsten powder, which is bound in a binder, the binder being a thermoplastic plastics material or a thermosetting plastics material or predominantly containing such a plastics material, and the molded body being produced by injection molding by the homogenized mixture, as an injection molding compound which has been prepared so as to be flowable, being filled into a mold (7) which molds the molded body by means of an injection molding machine,
**characterized in that**
the beam guide grid (4) is obtained **in that** movable mold cores (72) are introduced into the mold (7) before the filling of the injection molding compound, and **in that**, during the molding process, in order to ensure that the axes of the through-channels (40) are directed to a common focus point at an acute angle to one another, the mold cores (72) are arranged and formed in the mold in accordance with the orientation, the grid and the shape of the through-channels (40), which mold cores are held outside a wall portion of the mold (7) on a storage unit (8) and are removed from the through-channels (40) after filling and pre-solidification of the injection molding compound and before ejection of the molded body,
that the through-channels (40) widen conically from a beam incidence side to a beam exit side, the angle of conicity with respect to the central axis being at most 1°, and
**in that** the grid distance from center to center of the through-channels (40) is at most 2 mm and the thickness of the wall regions at the thinnest point thereof is at most 200 µm.

8. Arrangement of a large number of beam guide grids (4) of the design according to claim 7 arranged next to one another, in an optical system which has at least one x-ray source and in which the through-channels (40) are oriented toward the x-ray source with the central axes thereof.

9. Use of beam guide grids (4) of the design according to claim 7 or an arrangement according to claim 8 in a computer tomograph.

## Revendications

1. Procédé de fabrication d'une grille formant guide de rayonnements (4) fabriquée à partir d'un corps moulé pourvu d'une grille constituée de canaux traversants (40) entourés de zones de parois et d'un mélange de poudre métallique absorbant le rayonnement et d'agent liant, en particulier de poudre de tungstène et d'agent liant, le corps moulé étant fabriqué au moyen d'un moulage par injection et le mélange homogénéisé étant ainsi versé dans un outil de moule (7) formant le corps moulé sous la forme d'un composé d'injection préparé fluide au moyen d'une machine de moulage par injection,
**caractérisé en ce**
**que** des noyaux de moule (72) mobiles ont été introduits dans l'outil de moule (7) avant le versement du composé d'injection, et
**que** pendant le processus de moulage - afin de s'assurer que les axes des canaux traversants (40) sont dirigés à un angle aigu l'un par rapport à l'autre vers un point focal commun - les noyaux de moule (72) sont disposés et conçus dans l'outil de moule selon l'orientation, la grille et la forme des canaux traversants (40), lesquels sont maintenus sur une unité de palier (8) à l'extérieur d'une section de paroi de l'outil de moule (7), et sont retirés des canaux traversants (40) après le versement et une pré-solidification du composé d'injection et avant une éjection du corps moulé.

2. Procédé selon la revendication 1,
**caractérisé en ce**
**qu'**un matériau plastique thermoplastique ou duroplastique préchauffé et plastifié pour le processus d'injection est utilisé comme agent liant.

3. Procédé selon la revendication 2,
**caractérisé en ce**
**qu'**un polyéther cétone, en particulier un polyéther éther cétone (PEEK), est utilisé comme matériau plastique thermoplastique.

4. Procédé selon l'une des revendications précédentes,
**caractérisé en ce**
**que** le rapport de la poudre métallique, en particulier de la poudre de tungstène, à l'agent liant, en pourcentage en volume, se situe dans la plage de 30/70 à 98/2.

5. Procédé selon l'une des revendications précédentes,
**caractérisé en ce**
**que** les noyaux de moule (72), pour la préparation du processus de moulage, sont amenés dans la position de moulage au moyen d'une unité de réglage (9) déplaçant l'unité de palier (8) avec les noyaux de moule (72) et, après la pré-solidification, sont retirés des canaux traversants (40) au moyen de l'unité de réglage (9) déplaçant l'unité de palier (8).

6. Procédé selon l'une des revendications précédentes,
**caractérisé en ce**
**que** les noyaux de moule (72) sont insérés à travers des passages attribués individuellement dans la section de paroi de l'outil de moule (7) et sont retirés à travers ceux-ci, lesdits noyaux de moule étant maintenus flottants sur l'unité de palier (8) afin de compenser une composante de mouvement latérale dans les directions x et/ou y provoquée par un parcours oblique des canaux traversants (40) et des passages lors de l'introduction et du retrait.

7. Grille formant guide de rayonnements (4), laquelle est constituée d'un corps moulé comportant une grille de canaux traversants (40), lesquels sont alignés obliques avec leurs axes centraux à un angle aigu les uns par rapport aux autres vers un point focal, et comportant des zones de paroi entourant lesdits canaux de passage, absorbant les rayons X ou les rayons gamma, et formées à partir de poudre métallique, en particulier de poudre de tungstène, répartie de manière homogène et liée dans un agent liant, l'agent liant étant un matériau plastique thermoplastique ou duroplastique ou contenant majoritairement un tel matériau plastique et le corps moulé étant fabriqué par moulage par injection en versant le mélange homogénéisé sous la forme d'un composé d'injection préparé fluide à l'aide d'une machine de moulage par injection dans un outil de moule (7) formant le corps moulé,
**caractérisé en ce**
**que** la grille formant guide de rayonnements (4) est obtenue en ce que des noyaux de moule (72) mobiles ont été introduits dans l'outil de moule (7) avant le versement du composé d'injection et en ce que, pendant le processus de moulage - afin de s'assurer que les axes des canaux traversants (40) sont dirigés à un angle aigu les uns par rapport aux autres vers un point focal commun - les noyaux de moule (72) sont disposés et conçus dans l'outil de moule en fonction de l'orientation, de la grille et de la forme des canaux traversants (40), lesquels sont maintenus sur une unité de palier (8) à l'extérieur d'une section de paroi de l'outil de moule (7), et sont retirés des canaux traversants (40) après le versement et une pré-solidification du composé d'injection et avant une éjection du corps moulé,
**que** les canaux traversants (40) s'élargissent coniquement depuis un côté d'incidence des rayonnements jusqu'à un côté de sortie des rayonnements, l'angle de conicité par rapport à l'axe central étant d'au plus 1° et
**que** l'espacement de grille de centre à centre des canaux traversants (40) est d'au plus 2 mm et l'épaisseur des zones de paroi à leur point le plus mince est d'au plus 200 µm.

8. Agencement d'une pluralité de grilles formant guide de rayonnements (4) de la structure selon la revendication 7 disposées les unes à côté des autres, dans un système optique présentant au moins une source de rayons X, dans lequel les canaux traversants (40) sont alignés avec leurs axes centraux vers la source de rayons X.

9. Utilisation de grilles formant guide de rayonnements (4) de la structure selon la revendication 7 ou d'un agencement selon la revendication 8 dans un tomodensitomètre.
